# EUROPEAN PATENT APPLICATION

(11) **EP 4 834 370 A1**
(43) Date of publication of application: **04.11.2026**
(21) Application number: 24916728.9
(22) Date of filing: 24.12.2024
(51) Int. Cl.: A61K 47/52, A61K 47/64, A61K 47/58, A61K 31/675, B82Y 5/00, B82Y 40/00, A61P 35/00, A61P 29/00

(54) **NANO-ROBOT CAPABLE OF EFFICIENT AND TARGETED ENRICHMENT IN INFLAMMATORY LESION TISSUES AND USE THEREOF**

(30) Priority: 11.01.2024 CN 202410045509; 27.05.2024 CN 202410665010; 27.05.2024 CN 202410663937
(71) Applicant: Wuhan University Of Technology, Wuhan, Hubei 430000 (CN)
(72) Inventor: GUAN, Jianguo, Wuhan, Hubei 430000 (CN); LUO, Ming, Wuhan, Hubei 430000 (CN); YANG, Zili, Wuhan, Hubei 430000 (CN); GAO, Zhixue, Wuhan, Hubei 430000 (CN); PEI, Ziye, Wuhan, Hubei 430000 (CN)
(74) Representative: Schlich
(86) International application number: PCT/CN2024/141808
(87) International publication number: WO 2025/148681

(57) **Abstract**

The present disclosure relates to the field of micro-nano device technology, and in particular, to a nano-robot capable of efficient and targeted enrichment in inflammatory lesion tissues and use thereof. The nano-robot comprises a nanoparticle and at least two enzymes connected to the nanoparticle. At least one enzyme is used for driving the nano-robot in a physiological medium, and at least one other enzyme is used for sensing an inflammation lesion and controlling the motion direction of the nano-robot.

## Description

### TECHNICAL FIELD

The present disclosure relates to the technical field of microscale/nanoscale devices, and in particular to a nanorobot enabling efficient targeted accumulation in an inflammatory disease tissue, and use thereof.

### BACKGROUND

Tumor-targeted nanomedicine delivery *in vivo* involves five steps: blood circulation, accumulation in tumor tissue, penetration, cellular internalization, and intracellular drug release (collectively referred to as CAPIR). These five steps together determine the therapeutic efficacy of the nanomedicine against tumors. Existing nanomedicine delivery carriers cannot move autonomously, resulting in only 0.7% (median) of the nanomedicine reaching the tumor region, 0.0014% entering tumor cells, and only 2% of tumor cells coming into contact with the nanomedicine. Therefore, compared with traditional treatment methods, the therapeutic effect of tumor-targeted nanomedicine has not been significantly improved.

Chemotactic nanorobots (also known as nanomotors) are artificial microscale/nanoscale devices that, at the nanoscale, can convert other forms of energy into kinetic energy and can sense chemical concentration gradients in the environment to achieve directional movement. Owing to these properties, the nanomotors are expected to revolutionize the field of active targeted drug delivery as drug delivery carriers.

However, currently reported chemotactic nanorobots cannot be effectively driven within the blood circulatory system, and exhibit weak chemotactic sensitivity, making them unable to effectively sense the low-concentration chemical signals at actual lesion sites. As a result, the targeting efficiency at disease sites has not been significantly improved compared with that of inert carriers. These problems have greatly limited the application of nanorobots in the biomedical field.

### SUMMARY

In view of the technical problems existing in the background art, the present disclosure provides a nanorobot enabling efficient targeted accumulation in an inflammatory disease tissue, and use thereof, aiming to solve the technical problems of effective propulsion and precise targeted delivery of nanorobots as drug carriers in living organisms.

In a first aspect, the present disclosure provides a nanorobot enabling efficient targeted accumulation in an inflammatory disease tissue, including: a nanoparticle and two or more enzymes linked to the nanoparticle, where at least one of the enzymes is used for propelling the nanorobot in a physiological medium, and at least one other of the enzymes is used for sensing an inflammatory lesion site and controlling a movement direction of the nanorobot.

In the present disclosure, the at least one enzyme used for propelling the nanorobot in a physiological medium (hereinafter referred to as a propulsion enzyme) is an enzyme that uses an endogenous substance in the physiological medium as a substrate, where the endogenous substance includes but is not limited to urea, glucose (Glu), uric acid, an amino acid, lactose, and the like. For example, in some embodiments, urea is used as the substrate, and the propulsion enzyme is urease (URE), which provides a propelling force for the nanorobot through a catalytic reaction.

In the present disclosure, the at least one other enzyme used for sensing an inflammatory lesion site and controlling the movement direction of the nanorobot (hereinafter referred to as a chemotactic enzyme) is an enzyme that uses a biomarker released from the inflammatory lesion site as a substrate, where the biomarker includes but is not limited to hydrogen peroxide, glutathione, lactic acid, Glu, nucleic acid, adenosine triphosphate (ATP), hydrogen ions (H⁺), and the like. The released biomarkers vary with the type of inflammatory disease. For example, in some embodiments, the inflammatory disease is a tumor, and the chemotactic enzyme may be a catalase (CAT) that uses hydrogen peroxide released by tumor cells as a substrate. Based on the specific sensing function of the chemotactic enzyme for the lesion site, the steering function of the nanorobot is realized.

In the present disclosure, the nanoparticle includes but is not limited to elemental, oxide, or sulfide nanoparticles of a metal (including gold, silver, palladium, platinum, manganese, iron, cerium, titanium, aluminum, and zinc), inorganic non-metallic nanoparticles such as silicon dioxide, and organic nanoparticles (including dendrimers, liposomes, and polydopamine). The nanoparticle has a particle size of preferably 10 nm to 1000 nm, more preferably 20 nm to 200 nm. For example, in some embodiments, the nanoparticle is a gold nanoparticle. In addition, the nanoparticle can be of a hollow, solid, or porous structure, and a shape of the nanoparticle can be spherical, bowl-shaped, rod-shaped, or polyhedral. For example, in some embodiments, the nanoparticle is a solid sphere.

In the present disclosure, other functional substances can be linked to the nanoparticle to expand the functions of the nanorobot. Specifically, the other functional substances can be therapeutic drugs for the inflammatory disease tissue, and the therapeutic drugs include but are not limited to: chemotherapeutic drugs such as doxorubicin (DOX), cyclophosphamide, fluorouracil, gemcitabine, bleomycin, paclitaxel; photosensitizers such as chlorin E6 (Ce6), porphyrin mixture (e.g., Mthpc), porphyrin derivatives (e.g., BPMppa), phthalocyanines, methylene blue, texaphyrin; immunotherapeutic drugs such as CTLA-4 inhibitors, bispecific antibodies, monoclonal antibodies, antibody-drug conjugates; radiotherapeutic drugs such as sodium iodide, yttrium, ¹⁷⁷Lu-oxodotreotide, ²²³radium dichloride, ¹³¹I-tositumomab; and antioxidants such as glutathione peroxidase, superoxide dismutase (SOD), auranofin, curcumin, tetramethylpyrazine-curcumin hybrid. These therapeutic drugs are used such that they do not interfere with the synergistic action of the propulsion enzyme and chemotactic enzyme on the nanoparticle. For example, in some embodiments, the nanoparticle is simultaneously linked with an antioxidant for scavenging reactive oxygen species (ROS) at the inflammatory site. In other embodiments, the nanoparticle is simultaneously linked with a photosensitizer, thereby forming a nanorobot capable of conducting photodynamic therapy.

In a second aspect, the present disclosure provides a drug delivery system, including: the nanorobot provided by the present disclosure. The nanoparticle in the nanorobot can be loaded with a drug. It will be appreciated that the drug can be linked to the surface of the nanoparticle via a chemical bond, can be loaded within pores of the porous nanoparticle, or can be encapsulated inside the nanoparticle, and the mode of distribution is not limited. For example, it can be concentrated in a partial region of the nanoparticle, or dispersed on the entire surface of the nanoparticle, and the specific situation can be determined by comprehensively considering factors such as the type of nanoparticle and the mechanism of action of the drug. The propulsion enzyme provides a main motility for the nanorobot, and the chemotactic enzyme provides a chemotactic steering function, such that the nanorobot can effectively target the lesion site in the physiological medium, thereby achieving precise drug delivery.

In a third aspect, the present disclosure provides a medicament for targeted therapy of an inflammatory disease, where the medicament includes the above-mentioned nanorobot as a carrier, and can further include a pharmaceutically acceptable excipient. Experimental results show that, after the nanorobot is intravenously injected into a mouse, it can resist the random Brownian motion of nanoparticles, be effectively driven in a physiological medium (such as the blood circulation system or tumor microenvironment), and significantly improve the efficiency of targeting the lesion site.

In a fourth aspect, the present disclosure provides a medical device, including: the nanorobot provided by the present disclosure. Through a synergistic action of multiple enzymes, the nanorobot can autonomously move in a living body and efficiently target an inflammatory lesion site, and thus it can be used to fabricate microscale medical devices for interventional therapy in living organisms.

Compared with the prior art, the present disclosure has the following beneficial effects:
(1) Compared with existing chemotactic nanorobots, the nanorobot of the present disclosure innovatively utilizes the synergistic action of multiple enzymes to significantly improve the chemotactic sensitivity of the nanorobot and its targeting efficiency toward an inflammatory disease tissue, enabling the nanorobot to effectively accumulate at the inflammatory lesion site in a physiological medium. For example, compared with the traditional single-enzyme chemotactic nanorobots (such as CAT-driven chemotactic nanorobots), the chemotactic ability of the nanorobot is improved by more than 10-fold, and its targeting efficiency is improved by more than 20-fold; compared with an inert carrier, the targeting efficiency is improved by more than 200-fold.
(2) The nanorobot of the present disclosure has a wide range of applications and ultra-high plasticity. Owing to the modular design of the nanorobot structure provided by the present disclosure, the nanoparticle, functional substances, propulsion enzyme, and/or chemotactic enzyme can be replaced as needed without affecting its function, thereby enabling efficient targeting and multifunctional therapeutic strategies for different diseases and different lesion sites.
(3) The preparation method of the nanorobot of the present disclosure is simple and efficient, which is conducive to large-scale production.
(4) The nanorobot of the present disclosure shows desirable biosafety. Given its excellent targeting efficiency, the nanorobot can significantly reduce the dosage of a drug, thereby significantly reducing toxic side effects and significantly improving the therapeutic effect of the drug.

### BRIEF DESCRIPTION OF THE DRAWINGS

To explain the technical solutions in embodiments of the present disclosure more clearly, the accompanying drawings required in the embodiments will be described below in brief. Apparently, the following accompanying drawings show merely some embodiments of the present disclosure, and other drawings may be derived from these accompanying drawings by a person of ordinary skill in the art without creative efforts.
FIG. 1 shows a schematic diagram of the structure and propulsion mechanism of the nanorobot provided by the present disclosure.
FIG. 2 shows a schematic diagram of the structure and propulsion mechanism of the nanorobot in Example 1 of the present disclosure.
FIG. 3 shows a transmission electron microscope (TEM) image and an elemental mapping image of the nanorobot prepared in Example 2 of the present disclosure.
FIG. 4shows a movement trajectory image, plots of mean square displacement, and diffusion coefficient as a function of urea concentration, and a plot of diffusion coefficient change with different ratios of URE to CAT, for the nanorobot in Example 2 of the present disclosure in urea solutions of different concentrations.
FIG. 5shows a graph of the chemotactic ability test results of the nanorobot in Example 4 of the present disclosure.
FIG. 6 shows a graph of the results for *in vivo* targeting efficiency test of the nanorobot in Example 4 of the present disclosure.
FIG. 7 shows a graph of the results for chemotactic ability test of the nanorobot in Example 6 of the present disclosure.
FIG. 8 shows a schematic diagram of the structure and propulsion mechanism of the nanorobot in Example 7 of the present disclosure.
FIG. 9 shows a TEM image and an elemental mapping image of the nanorobot prepared in Example 7 of the present disclosure.
FIG. 10 shows a graph of the results for chemotactic ability test of the nanorobot in Example 7 of the present disclosure.
FIG. 11 shows a schematic diagram of the structure and mechanism of action of the nanorobot in Example 9 of the present disclosure.
FIG. 12 shows a TEM image (A) and an elemental mapping image (B) of the nanorobot in Example 10 of the present disclosure.
FIG. 13 shows a graph of the results for motility test of different nanorobots in a physiological concentration of urea solution in Example 11 of the present disclosure.
FIG. 14 shows a graph of the test results of targeting efficiency of the nanorobot to colon tissue in mice in Example 12 of the present disclosure.
FIG. 15 shows a graph of the therapeutic effect of the nanorobot on colitis in mice in Example 12 of the present disclosure.
FIG. 16 shows a graph of the results for *in vivo* safety evaluation of the nanorobot in Example 12 of the present disclosure.
FIG. 17 shows a schematic diagram of the structure and propulsion mechanism of the nanorobot in Example 13 of the present disclosure.
FIG. 18 shows a morphology and structure characterization image of the nanorobot prepared in Example 14 of the present disclosure.
FIG. 19 shows a comparison of the motility of different nanorobots in a physiological concentration of urea solution in Example 15 of the present disclosure.
FIG. 20 shows a graph of the targeting efficiency test results of the nanorobot for breast cancer in Example 16 of the present disclosure.
FIG. 21 shows a graph of the tumor inhibition effect test results of the nanorobot on tumor-bearing mice in Example 16 of the present disclosure;
FIG. 22 shows a graph of the results for biosafety test of the nanorobot during treatment of tumor-bearing mice in Example 16 of the present disclosure.
FIG. 23 shows a graph of the results for chemotactic ability test of the nanorobot in Example 17 of the present disclosure.
FIG. 24 shows a graph of the results for chemotactic ability test of the nanorobot in Example 18 of the present disclosure.

### DETAILED DESCRIPTION OF THE EMBODIMENTS

The embodiments of the present disclosure will be described in detail below with reference to the accompanying drawings. The following embodiments are used merely to clearly illustrate the technical solutions of the present disclosure. Therefore, these embodiments are merely exemplary and are not intended to limit the protection scope of the present disclosure.

Unless otherwise defined, all technical and scientific terms used herein have the same meanings as commonly understood by those skilled in the technical field of the present disclosure. The terms used herein are merely intended to describe the specific embodiments, rather than limit the present disclosure. The terms "comprise or include" and "have" in the specification, claims, and description of the accompanying drawings of the present disclosure and any variations thereof are intended to be non-exclusive.

In the specification and claims of the present disclosure, the terms "a or an" and "another" are only used to distinguish different objects, and shall not be construed as indicating or implying relative importance or implicitly indicating a specific order or primary and subordinate relationship of the indicated technical features. The term "more" means two or more (including two). The term "room temperature" means that the temperature can be within 4°C to 40°C. The term "and/or" is merely an association relationship describing associated objects, indicating that three relationships can exist, for example, A and/or B can represent: the situation where A exists alone, both A and B exist simultaneously, and B exists alone.

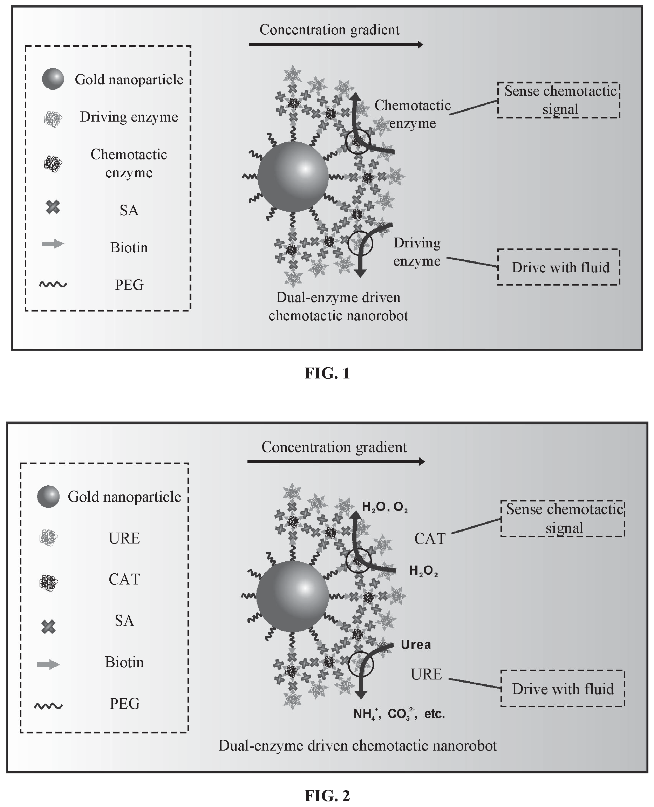

In order to solve the technical problems that existing nanorobots cannot be effectively driven in a physiological medium and have weak chemotactic sensitivity, and at the same time have low targeting efficiency for disease sites, the present disclosure provides a nanorobot capable of efficient targeted accumulation in an inflammatory disease tissue. As shown in FIG. 1, the nanorobot has a multi-enzyme structure, specifically including a nanoparticle and multiple enzymes linked to the nanoparticle, where at least one of the multiple enzymes is a propulsion enzyme and is used for propelling the nanorobot in a physiological medium, and at least one other of the multiple enzymes is a chemotactic enzyme and is used for sensing tumor cells at an inflammatory lesion site and controlling a movement direction of the nanorobot. Through the synergistic action of multiple enzymes, the chemotactic sensitivity and targeting efficiency of the nanorobot in the physiological medium are significantly improved.

In the present disclosure, the inflammatory disease includes but is not limited to tumors, enteritis, pneumonia, arthritis, nephritis and other diseases. For example, in some embodiments, the inflammatory disease is a tumor; and in other embodiments, the inflammatory disease is specifically enteritis.

In the present disclosure, the physiological medium includes but is not limited to blood, tumor microenvironment, interstitial fluid and the like. For example, in some embodiments, the physiological medium is blood. In other embodiments, the physiological medium is the tumor microenvironment.

In the present disclosure, the enzymes in the nanorobot can be biological enzymes and/or nanozymes, as long as they can drive the nanorobot or control the movement direction of the nanorobot through a catalytic reaction system in the physiological medium.

In the present disclosure, the nanoparticle includes but is not limited to gold nanoparticles, silica nanoparticles, liposomes, magnetic nanoparticles, polydopamine nanoparticles and the like. In specific embodiments, nanoparticles of different types and different particle sizes can be selected according to actual needs, where the particle size of the nanoparticle is preferably 10 nm to 1000 nm, and more preferably 20 nm to 200 nm. For example, in some embodiments, the nanoparticle is a gold nanoparticle of 90 nm to 100 nm.

In the present disclosure, the propulsion enzyme uses an endogenous substance in the physiological medium as a substrate, and provides a propelling force for the nanorobot through a catalytic reaction. The endogenous substance in the physiological medium includes but is not limited to urea, Glu, and the like. For example, in some embodiments, the propulsion enzyme is urease (URE), which uses urea in blood as a substrate to undergo a catalytic reaction and drive the nanorobot to move in the blood; preferably, when the nanorobot exhibits a diffusion coefficient of no less than 1.0 µm²/s at physiological urea concentrations in humans, it can effectively target inflammatory sites in the blood circulation system. In another example, when Glu is used as the endogenous substance, the propulsion enzyme may be glucose oxidase (GOx).

In the present disclosure, the chemotactic enzyme is an enzyme that uses a biomarker released from the inflammatory lesion site as a substrate, and based on the specificity of the chemotactic enzyme for the biomarker, the steering function of the nanorobot is realized, thereby accurately targeting the inflammatory lesion site. The biomarkers released vary with the type of inflammatory disease. For example, in some embodiments, for hydrogen peroxide released by tumor cells, the chemotactic enzyme is CAT.

In the present disclosure, the mode of linkage of the propulsion enzyme and the chemotactic enzyme on the nanoparticle can be any one of the following:
1) the propulsion enzyme and the chemotactic enzyme are linked layer-by-layer on a same side of the nanoparticle;
2) the propulsion enzyme and the chemotactic enzyme are linked in a same layer on the same side of the nanoparticle;
3) the propulsion enzyme and the chemotactic enzyme are linked layer-by-layer or in the same layer on the entire surface of the nanoparticle.

In cases 1) and 2), the assembly positions of the propulsion enzyme and the chemotactic enzyme relative to the nanoparticle are consistent, and they are linked only to a partial region of the nanoparticle, forming an asymmetric structure. In case 3), the two enzymes are directly linked to the entire surface of the nanoparticle, forming a relatively symmetric structure compared with thosecases 1) and 2). It should be noted that, since the chemical reactions of the two enzymes are not completely identical, the symmetry described in the present disclosure is not absolute symmetry, but a relatively symmetric state compared with the asymmetric structure of cases 1) and 2).

Experimental data of the present disclosure show that, when the propulsion enzyme and the chemotactic enzyme are linked layer-by-layer or in the same layer to the same side or to the entire surface of the nanoparticle, the chemotactic ability of the nanorobot can be significantly improved, with the only difference being different requirements for the ratio of the propulsion enzyme to the chemotactic enzyme. For example, when URE (propulsion enzyme) and CAT (chemotactic enzyme) are linked layer-by-layer on the same side, the propelling ability is the strongest when the ratio of URE to CAT is 1:1. When the two enzymes are linked in the same layer, the dosage of URE needs to be increased to achieve a similar technical effect. Preferably, the nanorobot with an asymmetric structure has a better effect.

In the present disclosure, the propulsion enzyme and the chemotactic enzyme can be linked to the nanoparticle via biotin-streptavidin interaction. For example, in some embodiments, the nanoparticle is a gold nanoparticle, and a method for linking the propulsion enzyme and the chemotactic enzyme to the gold nanoparticle includes: preparing asymmetric Au-PS (Au-polystyrene) eccentric particles, mixing the asymmetric Au-PS eccentric particles with hydroxyl-polyethylene glycol-thiol (HO-PEG-SH), then adding N,N-dimethylformamide (DMF) and thiol-polyethylene glycol-biotin (SH-PEG-Biotin), then adding streptavidin (SA), and finally adding biotinylated enzyme for reaction; where a molar ratio of the asymmetric Au-PS eccentric particles, the HO-PEG-SH, the SH-PEG-Biotin and the SA is 1:(1-50000):(1-50000):(1-5000), and preferably the molar ratio is 1:(2-10000):(2-10000):(2-1000).

In the present disclosure, the nanorobot further includes a therapeutic drug to achieve targeted treatment on the inflammatory disease tissue; where the therapeutic drug can be a chemotherapeutic drug, a radiotherapeutic drug, an immunotherapeutic drug, an antioxidant drug, a photosensitizer, and the like.

For example, in some embodiments of the present disclosure, the therapeutic drug is an antioxidant drug (i.e., an anti-inflammatory molecule), and the anti-inflammatory molecule is used alone or synergistically with the propulsion enzyme/chemotactic enzyme to scavenge ROS (reactive oxygen species) at the inflammatory site. A large amount of evidence shows that excessive ROS (including superoxide anion radical (•O₂⁻) and hydrogen peroxide (H₂O₂)) are closely related to the occurrence and development of inflammation. Therefore, further introducing a functional substance capable of scavenging ROS into the structure of the nanorobot is an effective strategy for treating inflammatory diseases.

In the present disclosure, the anti-inflammatory molecule is selected from biological enzymes, nanozymes (such as cerium dioxide), anti-inflammatory small molecule drugs, and other substances capable of scavenging ROS at the inflammatory site. For example, in some embodiments, the anti-inflammatory molecule is SOD, which catalyzes superoxide anions to generate hydrogen peroxide, thereby achieving treatment of inflammatory diseases by scavenging ROS at the inflammatory site. Meanwhile, if the chemotactic enzyme is CAT, the cascade reaction of SOD and CAT helps to further improve the ROS scavenging effect.

For example, in other embodiments of the present disclosure, the therapeutic drug is a photosensitizer, which is linked to the nanoparticle and induces cancer cell death upon light irradiation. As a small molecule substance, the photosensitizer has no effect on the enzyme-driven and enzyme-chemotactic capabilities of the nanorobot, and after the photosensitizer is simultaneously linked to the nanoparticle, the resulting nanorobot can achieve photodynamic therapy at the inflammatory site (such as a tumor). In the specific implementation process, the type and amount of the photosensitizer can be changed according to actual needs. For example, in some embodiments, the photosensitizer is Ce6.

It will be appreciated that one or more types of the therapeutic drug may be linked to the nanoparticle, and the mode of linkage and mode of distribution of the therapeutic drug on the nanoparticle are not limited. For example, it can be concentrated in a partial region of the nanoparticle, or dispersed on the entire surface of the nanoparticle. For example, when the therapeutic drug is an enzyme (such as SOD), it can be assembled with the propulsion enzyme and the chemotactic enzyme, layer-by-layer or in the same layer, on the same side or on the different sides of the nanoparticle, or it can be assembled with the propulsion enzyme and the chemotactic enzyme, layer-by-layer or in the same layer, on the entire surface of the nanoparticle.

In view of the excellent chemotactic sensitivity and targeting efficiency of the nanorobot provided by the present disclosure, the nanorobot may be used to prepare products such as medicaments for targeted treatment of different inflammatory diseases, drug delivery systems, and medical devices, and can be widely applied in the biomedical technical field.

The following lists some specific examples. It should be noted that the examples described below are exemplary and are only used to explain the present disclosure, and should not be understood as limitations of the present disclosure. If specific techniques or conditions are not indicated in an example, a process shall be conducted in accordance with the techniques or conditions described in literatures in the art or in accordance with a product specification. All reagents or instruments for which no manufacturer is specified are conventional commercially available products.

### Example 1

This example provided a nanorobot (referred to as the CAT/URE driven nanorobot), the structure and propulsion mechanism of which are shown in FIG. 2. The nanorobot used gold nanoparticle as the nanoparticle core, URE as the propulsion enzyme, and CAT as the chemotactic enzyme, and the URE and the CAT were assembled in a layer-by-layer manner on one side of the gold nanoparticles via polyethylene glycol, biotin, and SA, forming an asymmetric structure.

### Example 2

For the nanorobot in Example 1, this example provided a preparation method, including the following steps:
(1) Preparation of gold nanoparticles

Synthesis of 10 nm gold nanoparticles: 1 mM chloroauric acid solution was taken and heated with stirring in an oil bath to boiling, and after boiling, heating was continued for 10 min, then 38.8 mM sodium citrate aqueous solution was quickly added, and heating was continued for reaction for 10 min, after which heating was stopped.

Priming growth of gold nanoparticles: an appropriate volume of the 10 nm gold nanoparticle seed solution, an appropriate volume of 60 mM sodium citrate solution, and 142.5 mL of deionized water were added into a three-necked flask, heated and stirred at 90°C, and after the system was stabilized, an appropriate volume of 25 mM chloroauric acid solution was added in two portions, and after 30 min, the first growth of gold nanoparticles was completed.

Subsequent growth of gold nanoparticles: about 1/3 of the sample solution was taken out, an equal amount of deionized water was added to the original three-necked flask, after the temperature was stabilized, an appropriate volume of 60 mM sodium citrate solution was added again, after stabilization, an appropriate volume of 25 mM chloroauric acid solution was added in two portions, and the reaction was conducted for 30 min.

By controlling the number of growth steps, gold nanoparticles of different sizes could be obtained, and in this example, gold nanoparticles with a particle size of 90 nm were specifically selected.

### (2) Preparation of asymmetric Au-PS eccentric particles

One milliliter (1 mL) of 50 mg/mL potassium persulfate solution, 1 mL of 6 mg/mL sodium 4-styrenesulfonate solution, 2.5 mL of water, and 16.5 mL of ethanol were added into a three-necked flask, preheated at 70°C for 10 min, then 200 µL of a mixture (styrene: divinylbenzene = 200:1, v/v) was added. 2 min later, 3 mL of the gold nanoparticle solution was added, the reaction was continued for 4 h, and asymmetric Au-PS eccentric particles were collected by centrifugation.

### (3) Preparation of biotinylated URE and biotinylated CAT

The CAT solution was mixed with a biotinylation reagent, shaken uniformly at room temperature, washed 5 times with deionized water, and stored at 4°C in phosphate buffer (PB) solution for later use. The URE solution was mixed with a biotinylation reagent, shaken uniformly at room temperature, washed 5 times with deionized water, and stored at 4°C in PB solution for later use.

### (4) Preparation of the nanorobot

The asymmetric Au-PS eccentric particles obtained in step (2) were dispersed in 6 mL of ultrapure water, 2 mL of the Au-PS solution was taken, and 0.1 mL of HO-PEG-SH (10 mg/mL) solution was added and mixed with shaking; 1 mL of DMF was added to the sample, and the mixture was dispersed in 500 µL of ultrapure water, and 100 µL of SH-PEG-Biotin (10 mg/mL) solution was added, and after shaking, it was dispersed in PB solution (10 mM, pH = 7.4). 100 µL of the above solution was taken and 100 µL of SA solution (0.02 mg/mL) was added, mixed, and then dispersed in PB solution (10 mM, pH = 7.4); 100 µL of the above solution was taken and 100 µL of biotinylated CAT solution was added for reaction with shaking. Subsequently, coupling of SA and an equal amount of biotinylated URE was continued, and after the reaction was completed, the nanorobot was collected by centrifugation and washing. The used amount ratio of biotinylated CAT to biotinylated URE was 1:1.

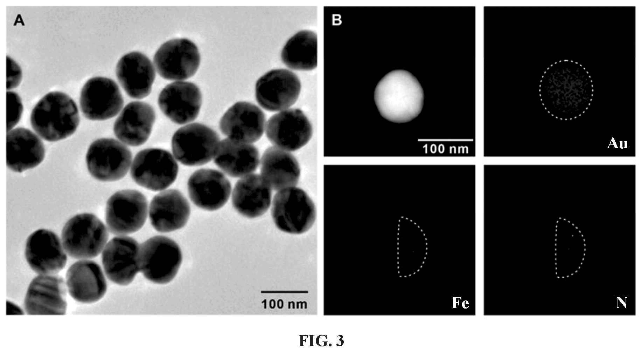

FIG. 3 shows a TEM image and an elemental mapping image of the nanorobot prepared in this example, where FIG. 3A shows a bright-field image of the chemotactic nanorobot in STEM mode of the TEM, and FIG. 3B shows the distribution of gold, nitrogen, and iron elements in dark-field and elemental mapping modes. According to the images, CAT and URE were uniformly distributed on one side of the gold nanoparticle, forming an asymmetric structure.

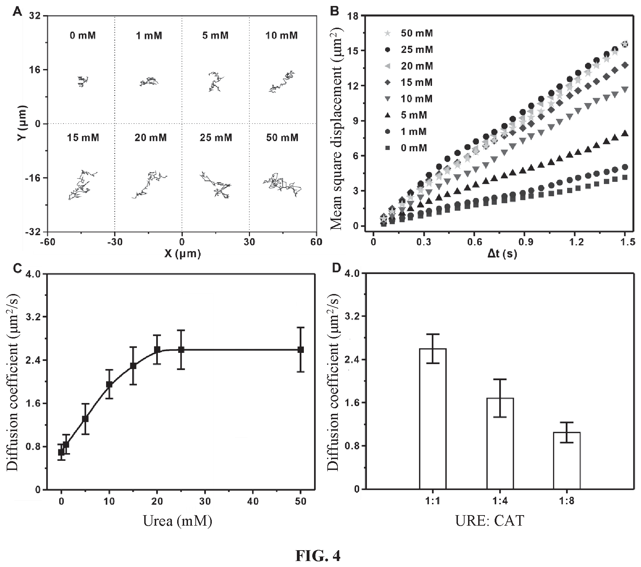

The nanorobots prepared in this example were placed in 0, 1, 5, 10, 15, 20, 25, and 50 mM urea solutions, respectively, to detect their motility in urea solutions of different concentrations. Their movement trajectories are shown in FIG. 4A, the variation of their mean square displacement (MSD = |r(t) - r(0)|², where r is the position at a certain time) is shown in FIG. 4B, and the variation of their diffusion coefficient (diffusion coefficient = MSD/4t) is shown in FIG. 4C. It can be seen from the figures that as the urea concentration increased, the movement distance of the nanorobot gradually increased, the slope of the mean square displacement and the diffusion coefficient also gradually increased, and the nanorobot could be effectively driven at physiological urea concentrations in humans (approximately 10 mM).

### Example 3

By referring to Example 2, nanorobots with different ratios of URE to CAT (URE: CAT = 1:(1-20)) were obtained in this example by changing the used amounts of biotinylated CAT and biotinylated URE, and the effects of the relative used amounts of URE and CAT on the motility of the nanorobots were detected.

In the CAT/URE driven nanorobot, URE provided the main motility, while hydrogen peroxide only provided the chemotactic steering function. When URE: CAT ratios were 1:1, 1:4, and 1:8, respectively, the test results of the motility of each nanorobot are shown in FIG. 4D, and the motility was strongest when URE: CAT was 1:1, and gradually reduced the URE proportion led to a significant decrease in motility.

### Example 4

Taking tumor, a disease with oxidative stress therein, as an example, it was demonstrated in this example that the targeting efficiency of the nanorobot shown in Example 1 toward tumor tissue under both *in vitro* and *in vivo* conditions, respectively, as follows:
(1) Preparation of control samples

In this example, an inert carrier, a URE-driven nanorobot, and a CAT-driven nanorobot were used as controls, and the preparation methods of the inert carrier, the URE-driven nanorobot, and the CAT-driven nanorobot followed the protocol in Example 2, with the only difference being that: both URE and CAT in the inert carrier were heat-inactivated, CAT in the URE-driven nanorobot was heat-inactivated, and URE in the CAT-driven nanorobot was heat-inactivated.

### (2) In vitro detection

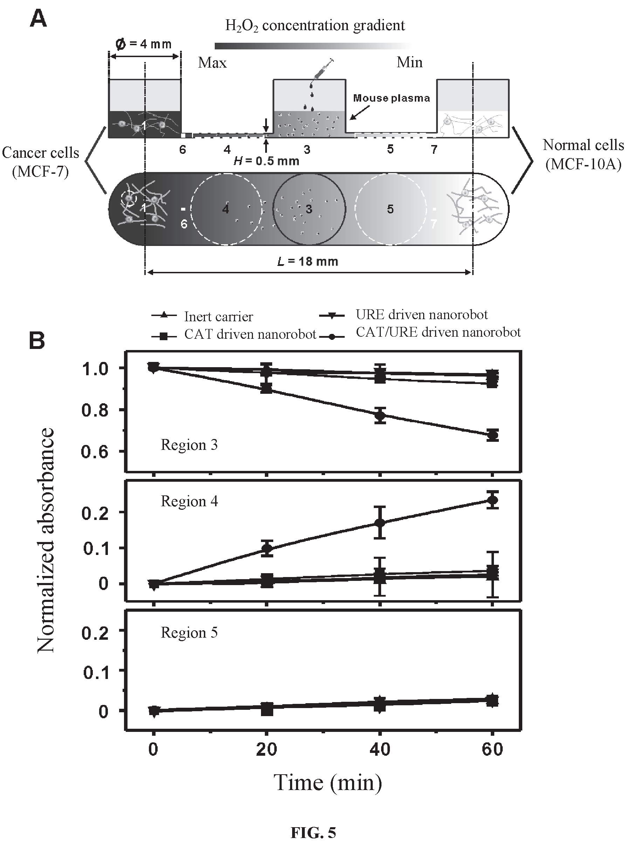

The nanorobots were placed in a 3D chip, and the change in absorbance was observed in Regions 3, 4, and 5, respectively, to determine the chemotactic ability of the nanorobots. The design of the 3D chip is shown in FIG. 5A. The left reservoir was filled with a gel matrix containing MCF-7 cells (tumor cells), while the right reservoir was filled with a gel matrix containing MCF-10A cells (normal cells), and the channels were filled with mouse plasma.

FIG. 5B shows a comparison of the chemotactic abilities of different nanorobots. It could be known from the test results that: the nanorobot provided by the present disclosure (prepared in Example 2) exhibited selectivity, could spontaneously move toward the cancer cell region but not toward normal cells, and the chemotactic ability of this nanorobot was significantly enhanced, being more than 10 times stronger than that of the traditional single-enzyme chemotactic nanorobot.

### (3) In vivo detection

The nanorobots were injected intravenously into tumor-bearing mice, and after 24 h, the mice were sacrificed, and the gold content in the tumor tissue was detected by inductively coupled plasma-mass spectrometry (ICP-MS), and the targeting efficiency of the nanorobots to the tumor was calculated.

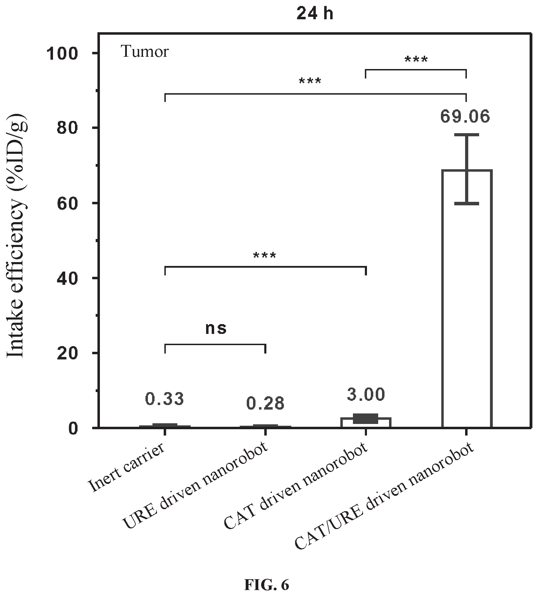

The targeting efficiencies of different nanorobots are shown in FIG. 6. The targeting efficiency of the nanorobot provided by the present disclosure (prepared in Example 2) was as high as 69.09% ID/g (targeting efficiency (% ID/g) = (gold content in tumor tissue measured by ICP-MS ÷ gold content of the injected nanorobots × 100%) ÷ weight of tumor tissue), which was 209 times that of the inert carrier, 246 times that of the URE-driven nanorobot having only the movement function, and 23 times that of the CAT-driven nanorobot.

### Example 5

This example provided a nanorobot, which differed from the nanorobot structure shown in Example 1 only in that: the URE and CAT were assembled in the same layer on one side of the gold nanoparticles via polyethylene glycol, biotin, and SA, thereby yielding a single-layer, dual-enzyme nanorobot.

### Example 6

For the nanorobot in Example 5, this example provided a preparation method, including the following steps:
(1) Preparation of gold nanoparticles: the same as step (1) in Example 2.
(2) Preparation of asymmetric Au-PS eccentric particles: the same as step (2) in Example 2.
(3) Preparation of biotinylated URE and biotinylated CAT: the same as step (3) in Example 2.
(4) Preparation of the chemotactic nanorobot: differed from step (4) in Example 2 only in that: after the nanoparticles were coupled with SA, biotinylated URE and biotinylated CAT were simultaneously mixed and coupled.

Based on the above method, nanorobots with different ratios of URE to CAT (URE: CAT = 1:(0.1-20)) were obtained by changing the used amounts of biotinylated CAT and biotinylated URE in step (4).

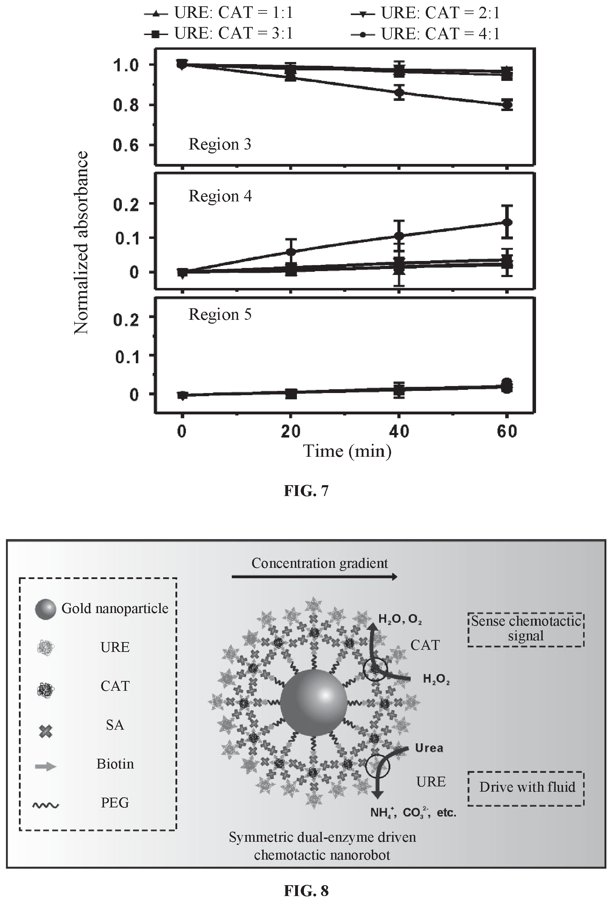

The single-layer dual-enzyme nanorobots of different ratios prepared in this example were subjected to *in vitro* detection using the 3D chip shown in FIG. 5A. The test results of the motility of the nanorobots with URE: CAT of 1:1, 2:1, 3:1 and 4:1, respectively, are shown in FIG. 7. It could be known from the test results that, by changing the ratio of URE to CAT to a suitable proportion, the single-layer dual-enzyme nanorobots were similar to the double-layer dual-enzyme nanorobots, they could not only selectively and spontaneously move toward the cancer cell region, but also significantly improve their chemotactic ability.

### Example 7

This example provided a nanorobot, the structure and propulsion mechanism of which are shown in FIG. 8. In the nanorobot, gold nanoparticle was used as the nanoparticle core, URE as the propulsion enzyme, and CAT as the chemotactic enzyme. The URE and the CAT were assembled in a layer-by-layer manner on the entire surface of the gold nanoparticles via polyethylene glycol, biotin, and SA, forming a relatively symmetric structure.

### Example 8

For the nanorobot in Example 7, this example provided a preparation method, including the following steps:
(1) Preparation of gold nanoparticles: the same as step (1) in Example 2.
(2) Preparation of biotinylated URE and biotinylated CAT: the same as step (3) in Example 2.
(3) Preparation of the chemotactic nanorobot.

The gold nanoparticles obtained in step (1) were dispersed in 6 mL of ultrapure water, 2 mL of the gold nanoparticle solution was taken, 100 µL of SH-PEG-Biotin (10 mg/mL) solution was added, and after shaking, it was dispersed in PB solution (10 mM, pH = 7.4). 100 µL of the above solution was mixed with 100 µL of SA solution (0.02 mg/mL), and the mixture was dispersed in PB solution (10 mM, pH = 7.4); 100 µL of the above solution was taken and 100 µL of biotinylated CAT solution was added for reaction with shaking. Subsequently, coupling of SA and biotinylated URE was continued, and after the reaction was completed, the nanorobot was obtained by washing. The used amount ratio of biotinylated CAT to biotinylated URE was 1:1.

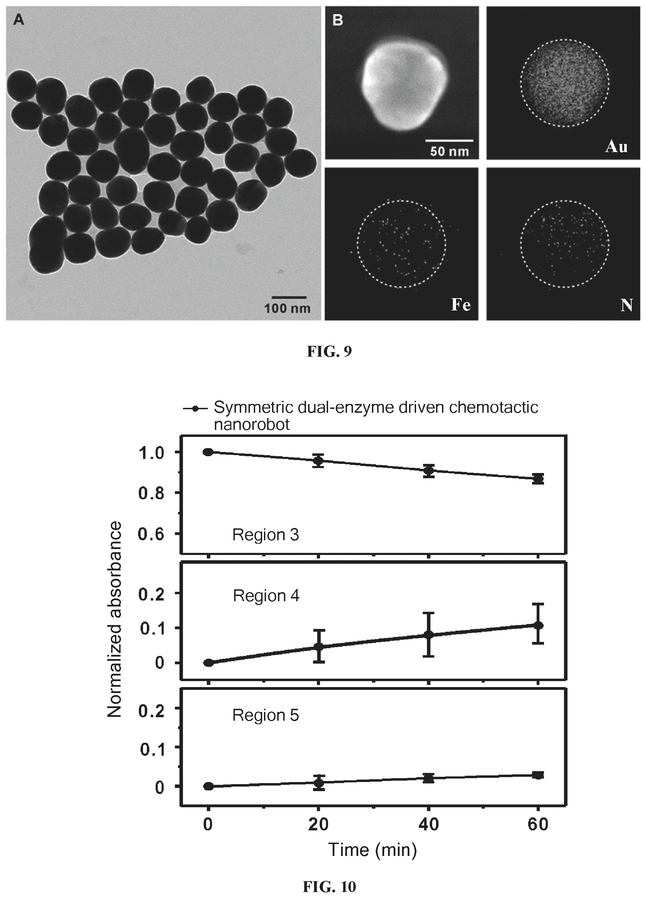

FIG. 9 shows a TEM image and an elemental mapping image of the nanorobot prepared in this example, where FIG. 9A shows a bright-field image of the chemotactic nanorobot in STEM mode of the TEM, and FIG. 9B shows the distribution of gold, nitrogen, and iron elements in dark-field and elemental mapping modes. According to the images, CAT and URE were distributed on both sides of the nanorobot, forming a relatively symmetric structure.

*In vitro* detection of the symmetric dual-enzyme driven nanorobot prepared in this example was conducted using the 3D chip shown in FIG. 5A. The result is shown in FIG. 10, and the chemotactic ability of this nanorobot was significantly enhanced, being more than 5 times stronger than that of the traditional single-enzyme nanorobot.

### Example 9

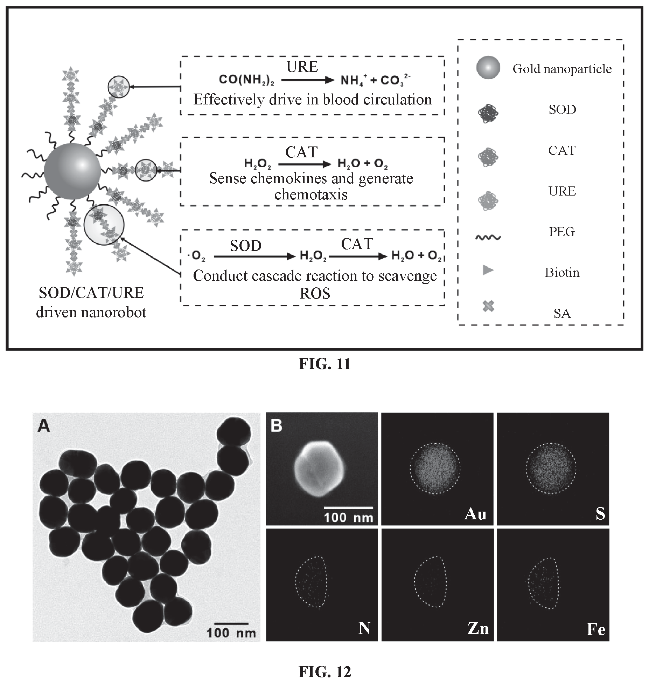

This example provided a nanorobot (referred to as the SOD/CAT/URE driven nanorobot), which, based on the nanorobot structure shown in Example 1, was simultaneously linked with an anti-inflammatory molecule, and its structure and propulsion mechanism are shown in FIG. 11. The nanorobot used gold nanoparticle as the nanoparticle core, URE as the propulsion enzyme, CAT as the chemotactic enzyme, and SOD as the anti-inflammatory molecule. The URE, the CAT, and the SOD were assembled in a layer-by-layer manner on one side of the gold nanoparticles via polyethylene glycol, biotin, and SA.

In the nanorobot structure of this example, the catalytic reaction between URE and urea was utilized to effectively drive the nanorobot under *in vivo* conditions (such as blood), the CAT was utilized to sense the hydrogen peroxide concentration gradient at the inflammatory site (i.e., the chemotactic source) to produce a chemotactic effect, and the synergistic action of URE and CAT enabled the nanorobot to accumulate efficiently and precisely at the inflammatory site, and then the cascade reaction of SOD and CAT enabled the ROS to be efficiently scavenged.

### Example 10

For the nanorobot in Example 9, this example provided a preparation method, including the following steps:
(1) Preparation of gold nanoparticles: gold nanoparticles with a particle size of 90 nm were obtained by referring to the method of step (1) in Example 2.
(2) Preparation of asymmetric Au-PS eccentric particles: the same as step (2) in Example 2.
(3) Preparation of biotinylated SOD, URE, and CAT: Referring to step (3) in Example 2.
(4) Preparation of the SOD/CAT/URE driven nanorobot.

The asymmetric Au-PS eccentric particles were dispersed in 5 mL of ultrapure water, 1 mL of the Au-PS solution was taken, and 0.1 mL of 10 mg/mL SH-PEG-Biotin solution was added and mixed with shaking; 1 mL of DMF was added to the sample, which was dispersed in 500 µL of deionized water, and 0.1 mL of 10 mg/mL methoxy poly(ethylene glycol)-thiol (mPEG-SH) solution was added, and after shaking, it was dispersed in PB solution (10 mM, pH = 7.4). 100 µL of the above solution was taken and 100 µL of SA solution (0.02 mg/mL) was added, mixed, and then dispersed in PB solution (10 mM, pH = 7.4). 100 µL of the above solution was taken and 100 µL of biotinylated SOD solution was added for reaction with shaking. By repeating the above coupling procedure, biotinylated CAT and URE were sequentially assembled onto the surface of the gold nanoparticles. After the reaction was completed, the SOD/CAT/URE driven nanorobot was obtained by washing. The used amount ratio of biotinylated SOD, CAT, and URE was 4:1:1.

FIG. 12 shows a TEM image and an elemental mapping image (scale bar 100 nm) of the SOD/CAT/URE driven nanorobot prepared in this example, where FIG. 12A shows a bright-field image of the SOD/CAT/URE driven nanorobot in STEM mode of the TEM, and FIG. 12B shows the distribution of gold, sulfur, nitrogen, iron, and zinc elements in dark-field and elemental mapping modes. According to the images, SOD, CAT, and URE were asymmetrically distributed on one side of the nanorobot, forming an asymmetric structure.

### Example 11

By referring to Example 10, SOD/CAT/URE nanorobots with different ratios of CAT to URE (CAT: URE = (1-20):1) were prepared in this example by changing the used amount ratio of biotinylated CAT and biotinylated URE in step (4).

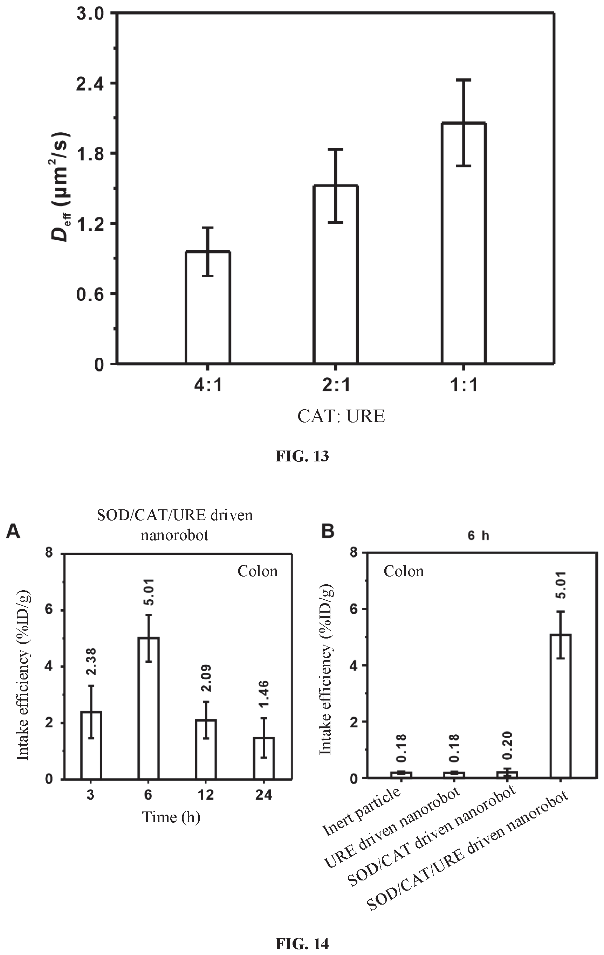

The motility of the SOD/CAT/URE driven nanorobots with different ratios of CAT to URE in urea solution at a physiological concentration was detected, respectively. Specifically: the nanorobots were placed in 10 mM urea solution, and their diffusion coefficients were tested to detect the influence of the relative used amounts of CAT and URE on the motility of the nanorobots. The results when the ratios of CAT to URE were 2:1, 4:1 and 1:1are shown in FIG. 13, and the motility was strongest when the ratio of CAT to URE was 1:1, and gradually reduced the URE proportion led to a decrease in motility.

### Example 12

With colitis, an inflammatory disease, as an example, it wasdemonstrated in this example that the *in vivo* targeting efficiency and therapeutic effect of the nanorobot shown in Example 9.

### (1) Preparation of control samples

In this example, inert particles, URE-driven nanorobots, and SOD/CAT-driven nanorobots were used as controls, and the preparation method of the control samples followed the protocol in Example 10, with the only difference being that: all of URE, CAT, and SOD in the inert particles were heat-inactivated, both CAT and SOD in the URE-driven nanorobots were heat-inactivated, and URE in the SOD/CAT-driven nanorobots was heat-inactivated.

### (2) Establishment of ulcerative colitis mouse model

In this example, the model was established by induction with dextran sulfate sodium (DSS) (2.5% w/v) for 7 days.

### (3) Detection of targeting efficiency

The SOD/CAT/URE driven nanorobots prepared in Example 10 and the control samples were separately dissolved in PBS solution to prepare different nanorobot injection solutions. Then the nanorobots were injected intravenously into the colitis mouse models at an injection volume of 200 µL and a dose of 1.5 mg/kg, the mice were sacrificed at 3, 6, 12 and 24 h, and the gold content in the colon tissue was detected by ICP-MS, thereby calculating the targeting efficiency of the nanorobots to colitis.

The test results are shown in FIG. 14. FIG. 14A shows the targeting efficiency in the nanorobots at different points of time, and FIG. 14B shows the targeting efficiency in different control groups at 6 h. It can be seen from FIG. 14 that the targeting efficiency of the SOD/CAT/URE driven nanorobot to colitis reached saturation at 6 h after intravenous injection, with a targeting efficiency of 5.01% ID/g, which was 28 times that of inert particles and 25 times that of the SOD/CAT-driven nanorobot.

### (4) Detection of therapeutic effect

The SOD/CAT/URE driven nanorobots prepared in Example 10 and the control samples were respectively dissolved in PBS solution to prepare nanorobot injection solutions. Then the injection solutions were injected intravenously into the colitis mice at an injection volume of 200 µL and a dose of 1.5 mg/kg, once every other day. After four injections, the mice were sacrificed, the colon, major organs (including the heart, liver, spleen, lung, and kidney), and blood were collected, and the colon length was measured and photographs were taken.

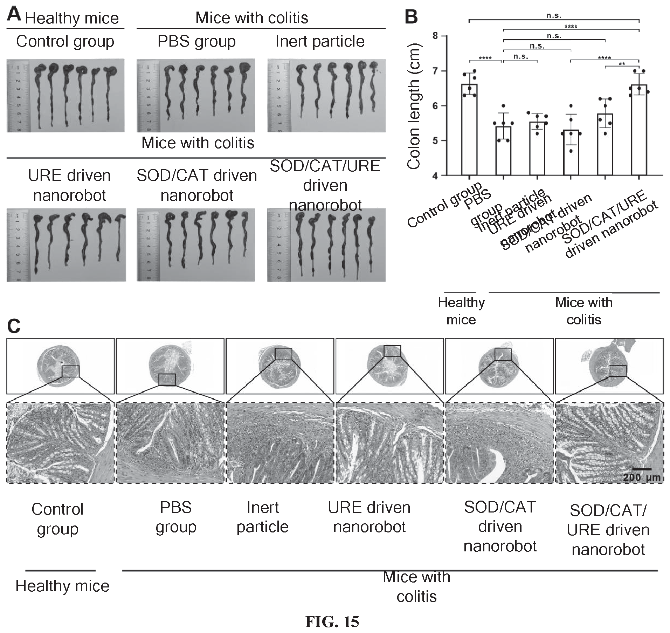

FIG. 15 shows the therapeutic effect of the SOD/CAT/URE driven nanorobot in colitis mice, where FIG. 15A is a colon photograph, FIG. 15B shows the colon length measurements, and FIG. 15C shows a pathological section of colon tissue (scale bar: 200 µm) stained with hematoxylin and eosin (H&E). According to FIG. 15, after treatment with the SOD/CAT/URE driven nanorobot, the colon length of the mice recovered to a normal state (6.6±0.3 cm), the typical inflammatory symptoms of colon shortening and colon damage were significantly improved, and the clinical symptoms of the colitis mice were effectively alleviated.

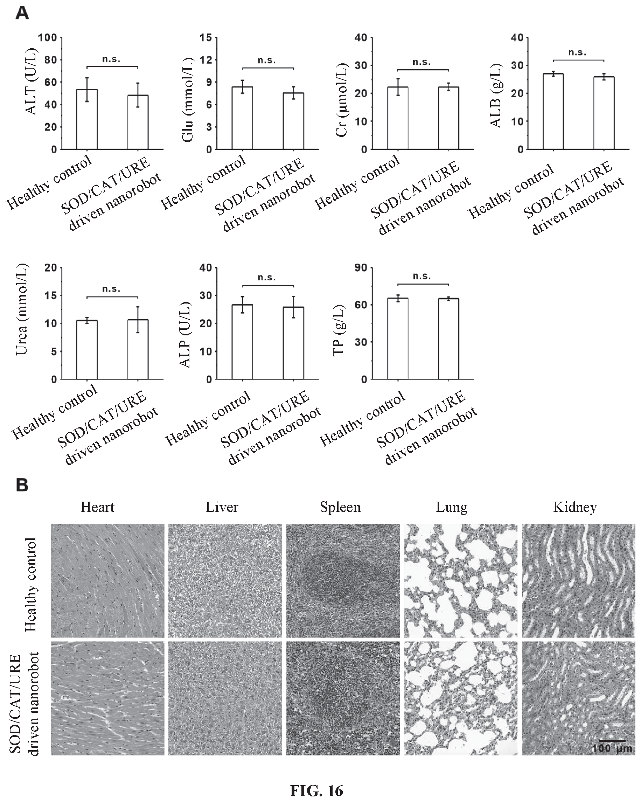

The blood and major organs of healthy mice and mice after treatment with the SOD/CAT/URE driven nanorobot were tested to conduct a biosafety evaluation. The test results are shown in FIG. 16, where FIG. 16A shows the results of blood biochemical parameter test of healthy mice and mice after the completion of nanorobot treatment, and FIG. 16B shows the H&E stained histological sections of major organs of healthy mice and mice after the completion of nanorobot treatment. It can be seen from FIG. 16 that no statistically significant difference was observed in blood biochemical parameters between nanorobot-treated mice and healthy mice, and all the H&E stained sections of the heart, liver, spleen, lung and kidney showed no obvious systemic toxicity.

### Example 13

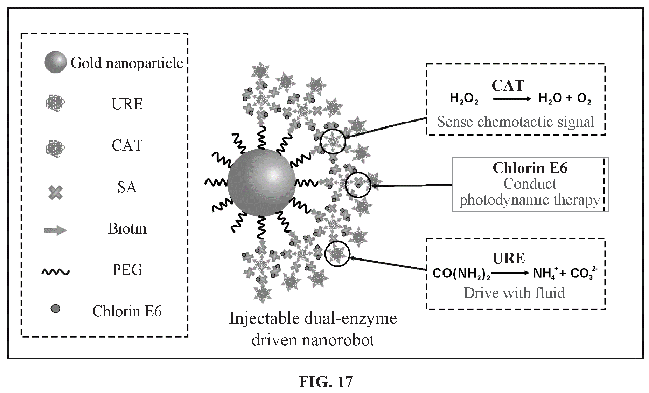

This example provided a nanorobot suitable for photodynamic therapy (referred to as the Ce6-CAT/URE driven nanorobot), which, based on the nanorobot structure shown in Example 1, was simultaneously linked with a photosensitizer, and its structure and propulsion mechanism are shown in FIG. 17. In the nanorobot, gold nanoparticle was usedas the nanoparticle core, URE as the propulsion enzyme, CAT as the chemotactic enzyme, and Ce6 as the photosensitizer. The URE and CAT were assembled in a layer-by-layer manner on one side of the gold nanoparticles via polyethylene glycol, biotin, and SA, and Ce6 was linked to the SA.

In the nanorobot structure of this example, CAT mediated chemotaxis by sensing hydrogen peroxide concentration gradients, and URE drove the nanorobot by catalyzing endogenous substrates in body fluids, thereby achieving efficient targeted accumulation of the nanorobot in an inflammatory disease tissue, and then treatment was conducted through Ce6-mediated photodynamic therapy.

### Example 14

For the photodynamic therapy nanorobot in Example 13, this example provided a preparation method, including the following steps:
(1) Preparation of gold nanoparticles: gold nanoparticles with a particle size of 90 nm were obtained by referring to the method of step (1) in Example 2.
(2) Preparation of asymmetric Au-PS eccentric particles: the same as step (2) in Example 2.
(3) Preparation of biotinylated URE and CAT: the same as step (3) in Example 2.
(4) Preparation of the photodynamic therapy nanorobot.

The asymmetric Au-PS eccentric particles were dispersed in 6 mL of ultrapure water, 2 mL of the Au-PS solution was taken, and 0.1 mL of HO-PEG-SH solution (10 mg/mL) was added and mixed with shaking; 1 mL of DMF was added to the sample, which was dispersed in 500 µL of ultrapure water, 100 µL of SH-PEG-Biotin solution (10 mg/mL) was added, and after shaking, it was dispersed in PB solution (10 mM, pH = 7.4). 100 µL of the above solution was taken and 100 µL of SA solution (0.02 mg/mL) was added, mixed, and then dispersed in a PB solution (10 mM, pH = 7.4). 100 µL of the above solution was taken and 100 µL of biotinylated CAT solution was added for reaction with shaking. Subsequently, coupling of SA and biotinylated URE was continued to obtain a dual-enzyme driven nanorobot. The used amount ratio of biotinylated URE to biotinylated CAT was 1:1.

0.2 µg of Ce6 was dissolved in 120 µL of dimethyl sulfoxide (DMSO), followed by the addition of 20 µL of 10 mM 1-ethyl-3-(3-dimethylaminopropyl)carbodiimide (EDC) and 10 µL of 10 mM N-hydroxysuccinimide (NHS). The mixture was shaken for half an hour, then the above dual-enzyme driven nanorobot was added, and shaken overnight. After the reaction was completed, the photodynamic therapy nanorobot was obtained by washing.

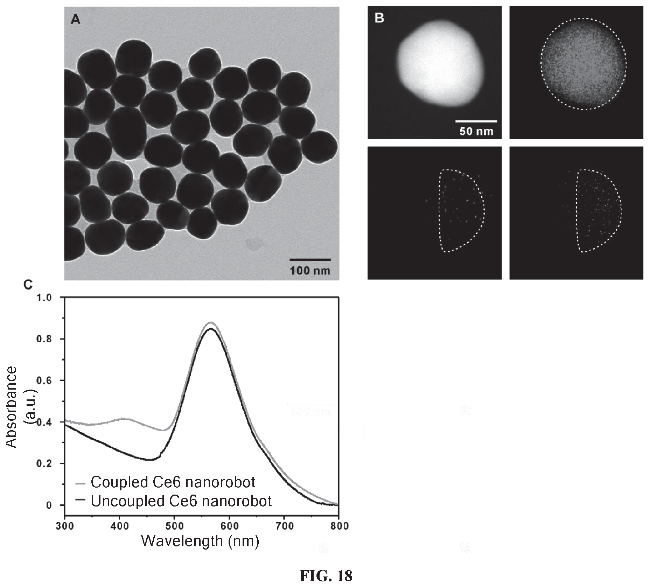

FIG. 18 shows the morphology and structure characterization images of the photodynamic therapy nanorobot prepared in this example. FIG. 18A and B are a TEM image and an elemental mapping image (scale bar: 100 nm) of the nanorobot, respectively. According to the images, CAT and URE were asymmetrically distributed on one side of the nanorobot, forming an asymmetric structure. FIG. 18C shows the ultraviolet absorption spectra of the photodynamic therapy nanorobot before and after Ce6 modification. The image shows that the coupled nanorobot has an obvious characteristic peak of Ce6 at 400 nm, indicating that Ce6 was successfully coupled.

### Example 15

By referring to Example 14, this example prepared photodynamic therapy nanorobots with different ratios of URE to CAT (URE: CAT = 1:(1-20)) by changing the used amount ratio of biotinylated CAT and biotinylated URE in step (4).

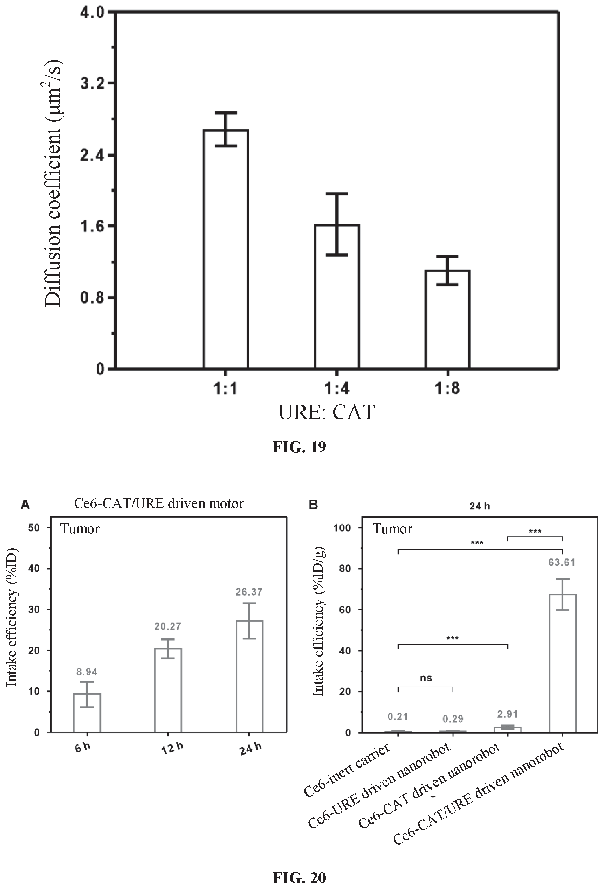

The diffusion coefficients of the photodynamic therapy nanorobots with different enzyme ratios in 10 mM urea solution were detected. The results when the ratios of URE to CAT were 1:1, 1:4, and 1:8 are shown in FIG. 19. The motility was strongest when the ratio of URE to CAT was 1:1, and gradually reducing the URE proportion led to a significant decrease in motility. It was also demonstrated that the nanorobot of the present disclosure could be effectively driven at physiological urea concentrations in humans (approximately 10 mM).

### Example 16

Taking breast cancer, an inflammatory disease, as an example, this example demonstrated the in vivo targeting efficiency and therapeutic effect of the nanorobot shown in Example 13.

### (1) Preparation of control samples

In this example, Ce6-inert carriers, Ce6-URE driven nanorobots, and Ce6-CAT driven nanorobots were used as controls, and the preparation method of the control samples followed the protocol in Example 14, with the only difference being that: both URE and CAT in the Ce6-inert carrier were heat-inactivated, CAT in the Ce6-URE driven nanorobot was heat-inactivated, and URE in the Ce6-CAT driven nanorobot was heat-inactivated.

### (2) Detection of targeting efficiency

The photodynamic therapy nanorobot prepared in Example 14 was resuspended in PBS solution and intravenously injected into 4T1 breast cancer tumor-bearing mice (the nanorobot dosage was 10 µg/mouse, among which the amount of Ce6 was about 0.0312 µg as quantified by ultraviolet spectrophotometry), the mice were sacrificed at 6 h, 12 h and 24 h, the gold content in tumor tissue was detected by ICP-MS, and the uptake efficiency was calculated. FIG. 20A shows an image of the tumor uptake efficiency over time. It can be seen from FIG. 20A that, as time increased, the tumor uptake of the photodynamic therapy nanorobot gradually increased, reaching a peak at 24 h, which was 26.37% ID.

The photodynamic therapy nanorobot prepared in Example 14 and its control samples were resuspended in PBS solution and intravenously injected into 4T1 breast cancer tumor-bearing mice (the dosage was 10 µg/mouse), and 24 h later, the mice were sacrificed, and the targeting efficiency of the nanorobots to the tumor was tested by ICP-MS. FIG. 20B shows the targeting efficiency results of the photodynamic therapy nanorobot and its control groups. It can be seen from FIG. 20B that the targeting efficiency of the Ce6-CAT/URE driven nanorobot was as high as 63.61% ID/g, which was 302 times that of the Ce6-inert carrier, 219 times that of the Ce6-URE driven nanorobot with only locomotion function, and 22 times that of the Ce6-CAT driven nanorobot.

The ordinate in FIG. 20A is the absolute uptake efficiency, and the calculation method thereof is specifically: the gold element content in the tumor tissue after grinding and digestion tested by ICP-MS and/or the gold element content of the injected nanorobot solution tested by ICP-MS. The uptake efficiency of the ordinate of FIG. 20B is the relative uptake efficiency, and the calculation method thereof is specifically: absolute uptake efficiency / tumor weight.

### (3) Detection of therapeutic effect

The photodynamic therapy nanorobot prepared in Example 14 and its control samples were respectively resuspended in PBS solution and then intravenously injected into 4T1 breast cancer tumor-bearing mice (the dosage was 10 µg/mouse). After 24 h, irradiation was conducted with a 660 nm laser at a power of 0.18 W/cm² for 10 min, and the operation was repeated once every three days for three repetitions. The mice were sacrificed 48 h later. The tumor size and body weight of the mice were recorded daily, and H&E staining was conducted on organs such as the heart, liver, spleen, lung and kidney.

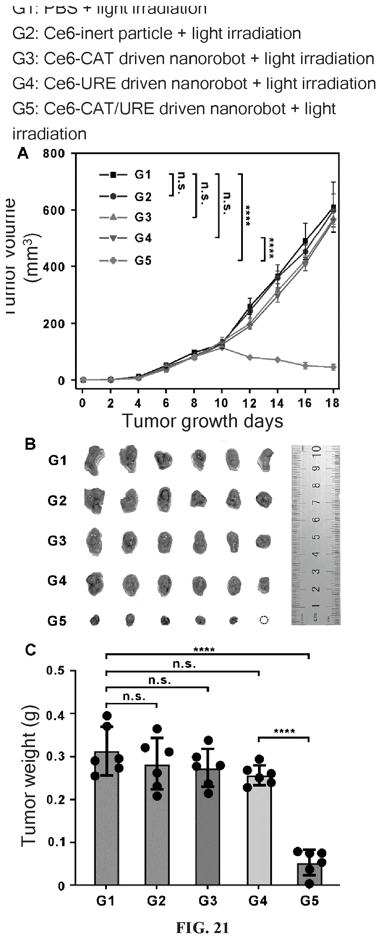

The tumor inhibition effect is shown in FIG. 21. FIG. 21A shows the change of mouse tumors over time, FIG. 21B shows photographs of different tissues, and FIG. 21C shows the tumor weight. It can be seen from FIG. 21 that, on the premise that the Ce6 loading amount of the Ce6-CAT/URE driven nanorobot was two orders of magnitude lower than that of other reported photodynamic nanorobots, its tumor inhibition effect was still very significant, and the tumor inhibition efficiency was as high as 92.7%.

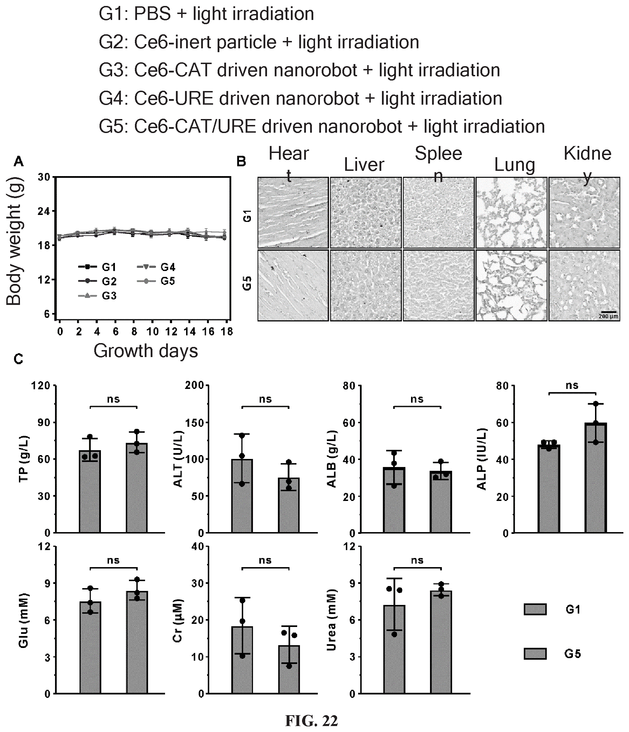

The biosafety is shown in FIG. 22. FIG. 22A shows the change of mouse body weight over time, FIG. 22B shows the tissue section images of the mice, and FIG. 22C shows the blood biochemical parameters of the mice. The above results indicated that the Ce6-CAT/URE driven nanorobot had desirable biosafety.

### Example 17

This example provided a nanorobot capable of being used for photodynamic therapy (referred to as the Ce6-GOx/URE driven nanorobot), which differed from the nanorobot shown in Example 13 only in that CAT was replaced with GOx. The Ce6-GOx/URE driven nanorobot was a nanorobot that produced enhanced chemotaxis toward a Glu concentration source.

The Ce6-GOx/URE driven nanorobot was prepared by referring to Example 14, and the following tests were conducted thereon:

By referring to Example 15, the diffusion coefficient of the Ce6-GOx/URE driven nanorobot in 10 mM urea solution was measured to be 2.63 µm²/s.

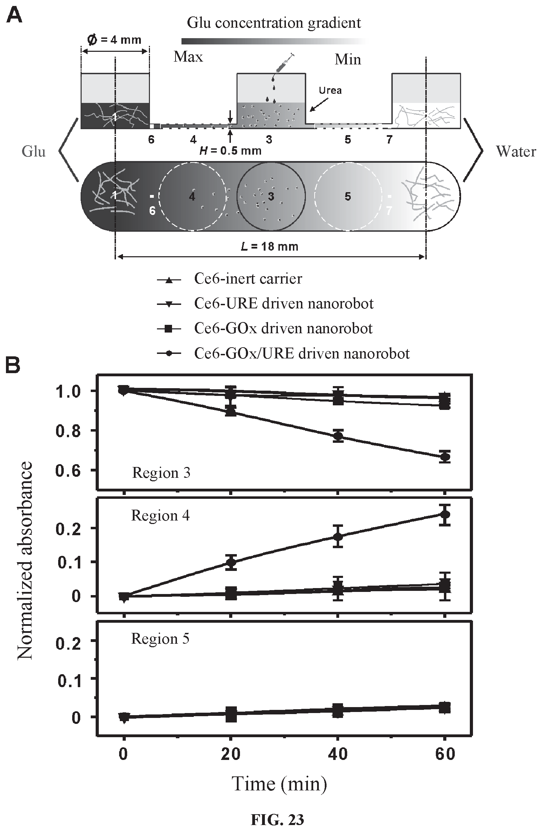

The Ce6-GOx/URE driven nanorobot was placed in a 3D chip, and the change in absorbance was observed in Regions 3, 4, and 5 to determine the chemotactic ability of the nanorobot. The design of the 3D chip is shown in FIG. 23A. The left reservoir was filled with a gel matrix containing 10 mM Glu, while the right reservoir was filled with a gel matrix containing deionized water, and the channels were filled with an aqueous urea solution (10 mM). FIG. 23B shows the chemotactic ability of the Ce6-GOx/URE driven nanorobot and its control samples (the preparation method of the control samples followed the protocol in Example 16). It could be known that: the Ce6-GOx/URE driven nanorobot had selectivity, could spontaneously move toward the high-concentration Glu region but not toward the other direction, and the chemotactic ability of this nanorobot was significantly enhanced, being more than 10 times stronger than that of the traditional single-enzyme chemotactic nanorobot.

### Example 18

This example provided a nanorobot suitable for photodynamic therapy (referred to as the Ce6-GGT/URE driven nanorobot), which differed from the nanorobot shown in Example 13 only in that CAT was replaced with γ-glutamyl transpeptidase (GGT). The Ce6-GGT/URE driven nanorobot was a nanorobot that produced enhanced chemotaxis toward a glutathione concentration source.

The Ce6-GGT/URE driven nanorobot was prepared by referring to Example 14, and the following tests were conducted thereon:

By referring to Example 15, the diffusion coefficient of the Ce6-GGT/URE driven nanorobot in 10 mM urea solution was measured to be 2.59 µm²/s.

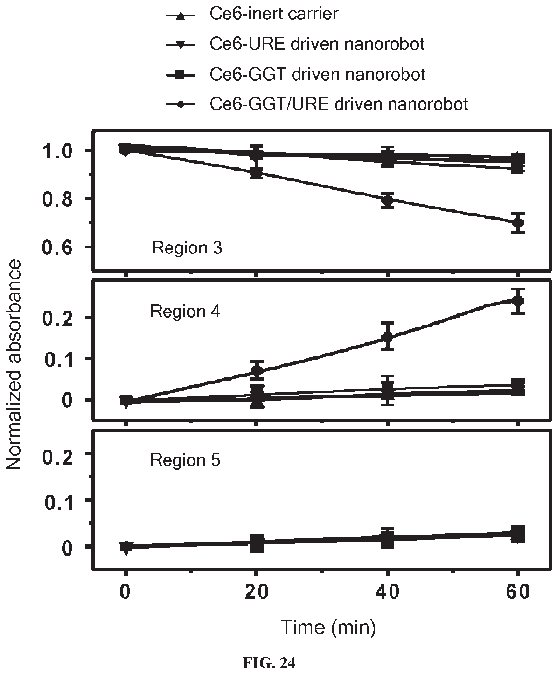

By referring to the test method in Example 17, the chemotactic ability of the Ce6-GGT/URE driven nanorobot was tested using a 3D chip, and the result is shown in FIG. 24. The test results showed that the Ce6-GGT/URE driven nanorobot had selectivity, could spontaneously move toward the high-concentration glutathione region but not toward the other direction, and the chemotactic ability of this nanorobot was significantly enhanced, being more than 10 times stronger than that of the traditional single-enzyme chemotactic nanorobot.

The various raw materials listed in the present disclosure, as well as the upper and lower limits and interval values of each raw material of the present disclosure, and the upper and lower limits and interval values of the process parameters, can all be implemented to achieve the technical effects of the present disclosure, and examples are not enumerated one by one here.

In summary, the nanorobot of the present disclosure using multiple enzymes has ultrahigh chemotactic sensitivity and ultrahigh targeting efficiency in living organisms, can overcome the complex environment in various physiological media (such as the blood circulation system and tumor microenvironment) and efficiently accumulate at the target inflammatory site. At the same time, the nanorobot can further carry different functional substances, such as photosensitizers, antioxidant drugs, chemotherapeutic drugs, etc., thereby enabling targeted treatment of various diseases through multiple strategies, and has excellent clinical application potential.

It should be noted that the present disclosure is not limited to the above embodiments. The above embodiments are merely examples, and embodiments that have the same composition and effect as the technical idea within a scope of the technical solutions of the present disclosure are included in the technical scope of the present disclosure. In addition, within the scope of the subject of the present disclosure, various variations that can be conceived by those skilled in the art to the embodiments and other embodiments constructed by combining some constituent elements of the embodiments are also included in the scope of the present disclosure.

## Claims

1. A nanorobot for efficient targeted accumulation in an inflammatory disease tissue, comprising: a nanoparticle and two or more enzymes linked to the nanoparticle, wherein at least one of the enzymes is used for propelling the nanorobot in a physiological medium, and at least one of the other enzymes is used for sensing an inflammatory lesion site and controlling a movement direction of the nanorobot.

2. The nanorobot according to claim 1, wherein the at least one enzyme used for propelling the nanorobot in the physiological medium is an enzyme that uses an endogenous substance in the physiological medium as a substrate, and the at least one of the other enzymes used for sensing the inflammatory lesion site and controlling the movement direction of the nanorobot is an enzyme that uses a biomarker released from the inflammatory lesion site as a substrate.

3. The nanorobot according to claim 2, wherein the endogenous substance is selected from the group consisting of glucose (Glu), lactose, a lipid, an amino acid, urea, uric acid, water, and any combination thereof; and the biomarker is selected from the group consisting of hydrogen peroxide, glutathione, lactic acid, Glu, nucleic acid, adenosine triphosphate (ATP), hydrogen ions (H⁺), and any combination thereof.

4. The nanorobot according to claim 1, further comprising: a therapeutic drug, wherein the therapeutic drug is selected from the group consisting of doxorubicin, cyclophosphamide, fluorouracil, gemcitabine, bleomycin, paclitaxel, chlorin E6, a porphyrin mixture photosensitizer, a porphyrin derivative photosensitizer, a phthalocyanine photosensitizer, methylene blue, a texaphyrin photosensitizer, a CTLA-4 inhibitor, a bispecific antibody immunotherapeutic drug, a monoclonal antibody immunotherapeutic drug, an antibody-drug conjugate, sodium iodide, yttrium, ¹⁷⁷Lu-oxodotreotide, radium chloride [²²³Ra], ¹³¹I-tositumomab, glutathione peroxidase, superoxide dismutase (SOD), auranofin, curcumin, and a tetramethylpyrazine-curcumin hybrid, and any combination thereof.

5. The nanorobot according to claim 4, wherein a mode of linkage of the therapeutic drug to the nanoparticle comprises: being linked to a surface of the nanoparticle, being loaded within pores of the porous nanoparticle, and being encapsulated inside the nanoparticle; and a mode of distribution of the therapeutic drug on the nanoparticle comprises: being concentrated in a partial region of the nanoparticle and being dispersed on the entire surface of the nanoparticle.

6. The nanorobot according to claim 1, wherein the nanoparticle is selected from the group consisting of an elemental, oxide, or sulfide nanoparticle of a metal, an inorganic non-metallic nanoparticle, and an organic nanoparticle, and the metal is selected from the group consisting of gold, silver, palladium, platinum, manganese, iron, cerium, titanium, aluminum, and zinc; a structure of the nanoparticle is selected from the group consisting of a hollow structure, a solid structure, and a porous structure, and a shape of the nanoparticle is selected from the group consisting of a spherical shape, a bowl shape, a rod shape, a polyhedral shape, and any combination thereof.

7. Use of the The nanorobot according to any one of claims 1 to 6 in manufacture of a medicament for treating an inflammatory disease, wherein the nanorobot improves a targeting efficiency and a therapeutic effect of the medicament.

8. The use according to claim 7, wherein the inflammatory disease is selected from the group consisting of a tumor, enteritis, and a combination thereof.

9. A medicament for targeted treatment of an inflammatory disease, wherein the medicament comprises the nanorobot according to any one of claims 1 to 3 as a delivery carrier.

10. A drug delivery system, comprising: the nanorobot according to any one of claims 1 to 3.

11. A medical device, comprising: the nanorobot according to any one of claims 1 to 3.
